# EUROPÄISCHE PATENTSCHRIFT

(11) **EP 2 114 860 B1**
(45) Veröffentlichungstag und Bekanntmachung des Hinweises auf die Patenterteilung: **29.09.2010**
(21) Anmeldenummer: 08709227.6
(22) Anmeldetag: 27.02.2008
(51) Int. Cl.: C07C 209/48, C07C 255/25, C07C 211/10, C07C 211/13, C07C 211/14

(54) **HERSTELLUNGSVERFAHREN FÜR ETHYLENAMINGEMISCHE**
PRODUCTION METHOD FOR ETHYLENEAMINE MIXTURES
PROCÉDÉ DE FABRICATION DE MÉLANGES ÉTHYLÉNAMINE

(30) Priorität: 01.03.2007 EP 07103297
(43) Veröffentlichungstag der Anmeldung: 11.11.2009
(73) Patentinhaber: BASF SE, 67056 Ludwigshafen (DE)
(72) Erfinder: DAHMEN, Kirsten, 67251 Freinsheim (DE); OFTRING, Alfred, 67098 Bad Dürkheim (DE); BAUMANN, Katrin, 68529 Mannheim (DE); HUGO, Randolf, 67246 Dirmstein (DE); HAHN, Thilo, 67292 Krichheimbolanden (DE); MELDER, Johann-Peter, 67459 Böhl-Iggelheim (DE)
(86) Internationale Anmeldenummer: PCT/EP2008/052337
(87) Internationale Veröffentlichungsnummer: WO 2008/104552

(56) Entgegenhaltungen:
- US-A- 2 429 876
- US-A- 2 436 368
- US-A- 5 869 653
- US-A1- 2002 058 842
- DATABASE WPI Week 200315 Derwent Publications Ltd., London, GB; AN 2003-151437 XP002479252 & JP 2002 338536 A (KOEI CHEM IND CO LTD) 27. November 2002 (2002-11-27)
- S. A. GAMAGE ET AL: "Dicationic Bis(9-methylphenazine-1-carboxamides): Relationships between biological activity and linker chain structure for a series of potent topoisomerase targeted anticancer agents" JOURNAL OF MEDICINAL CHEMISTRY., Bd. 44, 2001, Seiten 1407-1415, XP002479251 USAMERICAN CHEMICAL SOCIETY. WASHINGTON.

## Beschreibung

Die Erfindung betrifft ein Verfahren zur Herstellung eines Ethylenamingemisches durch Hydrierung eines Aminonitrilgemisches an einem Katalysator. Aus dem erhaltenen Ethylenamingemisch können die einzelnen Ethylenamine gegebenenfalls isoliert werden.

Es ist generell bekannt, dass Nitrile in Gegenwart von Katalysatoren zu den entsprechenden Aminen hydriert werden können. In Abhängigkeit von den gewählten Reaktionsparametern liefern die bekannten Verfahren die gewünschten Produkte, beispielsweise primäre Amine als Hauptprodukt und sekundäre und tertiäre Amine als Nebenprodukte.

Für Verfahren zur Herstellung von Aminen durch Hydrierung von Nitrilen ist zudem bekannt, dass ein gewisser Anteil an Ammoniak die Selektivität der Hydrierung zu primären Aminen begünstigt und die Bildung von sekundären und tertiären Aminen unterdrückt. Allerdings verursacht die Hydrierung in Gegenwart von Ammoniak zusätzlichen technischen Aufwand, der mit der Abtrennung aus dem Produktstrom, der Aufarbeitung und der eventuellen Rückführung des Ammoniaks verbunden ist. Darüber hinaus können höhere Drücke bei der Hydrierung erforderlich sein, da der Partialdruck des Ammoniaks berücksichtigt werden muss.

So kann durch Hydrierung von Aminoacetonitril (AAN) als Hauptprodukt Ethylendiamin (EDA) hergestellt werden, welches ein Ausgangsstoff beispielsweise für die Synthese von Komplexbildnern oder Bleichaktivatoren ist, die unter anderem als Waschmittel- oder Reinigungsadditive Verwendung finden. Analog liefert die Hydrierung von Iminodiacetonitril (IDAN) als Hauptprodukt Diethylentriamin (DETA). Jedoch fallen bei der Hydrierung von AAN bzw. IDAN immer auch DETA bzw. EDA als Nebenprodukte an.

Im Stand der Technik sind zahlreiche Verfahren zur Hydrierung der α-Aminonitrile Aminoacetonitril (AAN) und Iminodiacetonitril (IDAN) oder von β-Aminonitrilen beschrieben. So ist es bekannt, dass die Hydrierung von β-Aminonitrilen in aller Regel problemlos verläuft, während die Hydrierung von α-Aminonitrilen mit dem Auftreten von zahlreichen Nachteilen verbunden ist, wie der Hydrogenolyse der C-CN-Bindung oder der R₂N-C-Bindung. ""Handbook of Heterogeneous Catalytic Hydrogenation for Organic Synthesis, S. 213 - 215" zeigt die Problematik der Hydrierung von α-Aminonitrilen anhand von α-Alkylaminonitrilen oder cyclischen α-Aminonitrilen im Vergleich zu β-Aminonitrilen auf. Die bekannten Stabilitätsprobleme der α-Aminonitrile sind vermutlich der Hauptgrund dafür, warum bis heute nur die Hydrierung der α-Aminonitrile AAN oder IDAN zu EDA (Ethylendiamin) bzw. DETA (Diethylentriamin) genauer beschrieben wird. Großtechnisch werden EDA oder DETA jedoch durch die nachfolgend beschriebenen EDC- oder MEA-Verfahren hergestellt. Für höhere α-Aminonitrile ist eine entsprechende Hydrierung jedoch nicht bekannt.

In DE-A 3 003 729 wird ein Verfahren zum Hydrieren von aliphatischen Nitrilen, Alkylenoxynitrilen und Alkylenaminonitrilen zu primären Aminen in Anwesenheit eines Lösungsmittelsystems an einem Kobalt- oder Rutheniumkatalysator beschrieben. Das verwendete Lösungsmittelsystem enthält neben Wasser und Ammoniak einen Ether oder Polyether. Die als Edukte einsetzbare Alkylenaminonitrile oder Alkylenoxynitrile sind jeweils über komplexe allgemeine Formeln definiert. Unter anderem sind als konkrete Verbindungen oder Beispiele, die zum entsprechenden Diamin hydriert werden können, Ethylendiamindipropionitril (EDDPN; auch als N,N'-Bis(cyanoethyl)-ethylendiamin bezeichnet) oder 3,3'-(Ethylendioxy)-dipropionitril aufgeführt. DE-A 3 003 729 offenbart hingegen keinen Hinweis auf die Verwendung von Einzelverbindungen wie AAN oder von EDA-Derivaten mit Cyanomethylsubstituenten, wie Ethylendiamindiacetonitril (EDDN) oder Ethylendiaminmonoacetonitril (EDMN). Letztgenanntes fällt zudem nicht unter die allgemeine Definition von Alkylenaminonitrilen gemäß diesem Dokument.

EP-A 0 382 508 beschreibt ein Verfahren zur chargenweisen Herstellung von nicht-cyclischen, aliphatischen Polyaminen durch Hydrierung von nicht-cyclischen, aliphatischen Polynitrilen in flüssiger Phase an Raney-Kobalt-Katalysatoren, vorzugsweise in Gegenwart von wasserfreiem Ammoniak. Hierbei wird eine Polynitrillösung einer Reaktionszone zugeführt, die den Raney-Kobalt-Katalysator in einer im Wesentlichen sauerstofffreien Atmosphäre enthält. Während des gesamten Reaktionszeitraums wird die Polynitrillösung mit einer Rate zugeführt, die nicht größer ist als die maximale Rate, mit der das Polynitril mit dem Wasserstoff in der Reaktionszone reagiert. Es wird weiterhin ein Reaktionsparameter K benannt, welcher zur Bestimmung der volumetrischen Zufuhrrate geeignet ist. Das beschriebene Verfahren beschränkt sich auf die Herstellung von Polyaminen aus Polynitrilen, wie Iminodiacetonitril (IDAN), Nitrilotriacetonitril (NTAN) oder weitere Verbindungen mit 2 oder mehr Cyanogruppen. Die Umsetzung von Verbindungen mit einer Cyanogruppe, wie AAN zu EDA, wird jedoch nicht beschrieben.

EP-A 212 986 betrifft ein weiteres Verfahren, bei dem aliphatische Polynitrile in Gegenwart eines im Eduktstrom enthaltenen, flüssigen primären oder sekundären Amines an einem granularen Raney-Kobalt-Katalysators zu den entsprechenden Polyaminen hydriert werden können. Unter anderem ist die zwingend vorhandene Aminokomponente EDA neben zahlreichen weiteren primären oder sekundären Aminen aufgeführt.

Weiterhin offenbart dieses Dokument konkret, dass IDAN zu DETA hydriert werden kann.

DE-A 1 154 121 betrifft ein Verfahren zur Herstellung von Ethylendiamin, wobei die Edukte Blausäure, Formaldehyd, Ammoniak und Wasserstoff in Gegenwart eines Katalysators in einem so genannten Eintopf-Verfahren zur Reaktion gebracht werden. Sowohl der Ammoniak als auch der Wasserstoff werden im molaren Überschuss gegenüber den in äquimolaren Mengen vorliegenden weiteren Edukten Blausäure und Formaldehyd eingesetzt. In diesem Verfahren wird also das in situ gebildete AAN nicht isoliert, sondern direkt mit Wasserstoff weiter umgesetzt. Nachteilig an diesem Verfahren ist, dass das gewünschte Produkt (EDA) relativ unselektiv in kleinen Mengen anfällt.

US-A 3,255,248 beschreibt ein Verfahren zur Hydrierung von organischen Stickstoff-Kohlenstoff-Verbindungen, die vorzugsweise Nitro-, N-Nitroso, Isonitroso, Cyano oder mit Aromaten substituierte Aminogruppen aufweisen, zu den entsprechenden Aminen in flüssiger Phase unter Verwendung eines gesinterten Katalysators aus Kobalt oder Nickel. Hierbei wird das Edukt entweder alleine oder in Gegenwart eines Lösungsmittels, wie beispielsweise Wasser, Tetrahydrofuran, Methanol, Ammoniak oder das gebildete Reaktionsprodukt, gemeinsam mit dem Wasserstoff auf den Katalysator hinuntergerieselt. Sofern am Stickstoffatom ungesättigte Verbindungen, wie Cyanogruppen, hydriert werden, wird die Gegenwart von Ammoniak bei der Umsetzung empfohlen. Dies wird in Beispiel 1 dieses Patents verdeutlicht, wo Aminoacetonitril in Form einer wässrigen Lösung mit flüssigem Ammoniak, aber ohne sonstiges Lösungsmittel auf den gesinterten Katalysator gerieselt wird.

EP-A 1 209 146 betrifft ein weiteres Verfahren zur kontinuierlichen Hydrierung von Nitrilen zu primären Aminen, wobei die jeweiligen Nitrile in flüssiger Phase an einem suspendierten, aktivierten Raney-Katalysator auf Basis einer Legierung aus Aluminium eingesetzt werden und die Reaktion in Abwesenheit von Ammoniak und basischen Alkali- oder Erdalkaliverbindungen durchgeführt wird. Als Nitrile können neben vielen anderen auch AAN, IDAN, EDTN, EDDPN oder Ethylendiaminmonopropionitril (EDMPN) zu den entsprechenden Ethylenaminen umgesetzt werden.

EP-B 0 913 388 betrifft ein Verfahren zum katalytischen Hydrieren von Nitrilen, das den Kontakt des Nitrils mit Wasserstoff in Gegenwart eines Kobaltschwammkatalysators bei Bedingungen für die Durchführung der Umwandlung der Nitrilgruppe in das primäre Amin umfasst. Der Kobaltschwammkatalysator ist zuvor mit einer katalytischen Menge von Lithiumhydroxid behandelt worden und das Verfahren wird in Gegenwart von Wasser durchgeführt. Als Nitrile eignen sich aliphatische Nitrile mit 1 bis 30 Kohlenwasserstoffatomen, unter anderem auch β-Aminonitrile wie Dimethylaminopropionitril. Ein weiteres Verfahren zur Herstellung von Polyaminen aus den entsprechenden Polynitrilen wird in DE-A 27 55 687 offenbart. In diesem Verfahren wird die Hydrierung an einem Hydrierungskatalysator in Tablettenform in Gegenwart eines den Zerfall des Katalysators inhibierenden Stabilisators durchgeführt. Als Polynitril kann unter anderem Ethylendiamindipropionitril (EDDPN) verwendet werden. Als Stabilisator eignet sich unter anderem EDA.

US-A 2006/0041170 betrifft ein Verfahren zur Herstellung von TETA, insbesondere von TETA-Salzen, und deren Verwendung als Arzneimittel. In diesem mehrstufigen Verfahren wird zunächst EDDN hergestellt. Anschließend wird EDDN mit Benzaldehyd unter Ausbildung eines (cyclischen) Imidazolidin-Derivates umgesetzt. Diese cyclische Verbindung, die zwei Cyano-Gruppen aufweist wird reduziert, beispielsweise durch Umsetzung mit Wasserstoff, wobei die entsprechende cyclische Diamino-Verbindung erhalten wird. Diese Diamino-Verbindung wird wiederum in Gegenwart einer Säure hydrolysiert unter Erhalt des entsprechenden TETA-Salzes. In einer alternativen Ausführungsform wird die cyclische Diamin-Verbindung ebenfalls mit Benzaldehyd umgesetzt unter Ausbildung der entsprechenden Diimin-Verbindung, die anschließend wiederum in Gegenwart einer Säure unter Erhalt des entsprechenden TETA-Salzes hydrolysiert wird. Als weitere Verfahrensalternative wird in diesem Dokument die Umsetzung von EDDN mit Boc-Schutzgruppen (Tertiär-Butoxy-Carbonyl-Gruppen) beschrieben. Das dabei erhaltene, zweifach mit Boc-Schutzgruppen geschützte EDDN-Derivat wird anschließend unter Erhalt des entsprechenden geschützten TETA-Derivates hydriert. Die Entfernung der Boc-Schutzgruppen erfolgt durch saure Hydrolyse unter Erhalt des entsprechenden TETA-Salzes. Nachteilig an diesem in US-A 2006/0041170 beschriebenen Verfahren ist insbesondere, dass es sich um ein mehrstufiges Hydrierverfahren handelt, bei dem das eingesetzte Edukt EDDN zuerst chemisch derivatisiert werden muss, um die Hydrierung durchzuführen. Weiterhin ist nachteilig, dass zunächst TETA als Salz und nicht in der freien Basenform anfällt.

Die Herstellung von höheren Ethylenaminen wie Triethylentetraamin (TETA) oder Tetraethylenpentamin (TEPA) durch direkte Hydrierung der entsprechenden α-Aminonitrile ist bis jetzt noch nicht beschrieben worden. Höhere Ethylenamine wie TE-TA oder TEPA werden (großtechnisch) nach anderen Verfahren hergestellt.

EP-A 222 934 betrifft ein Verfahren zur Herstellung von höheren Alkylenpolyaminen durch Umsetzung eines vicinalen Dihaloalkans mit einem Überschuss an Ammoniak in wässriger Phase unter Zugabe einer starken Base, wobei ein Imin-Zwischenprodukt gebildet wird, das anschließend mit einem Alkylenpolyamin unter Ausbildung des höheren Alkylenpolyamins umgesetzt wird. Als vicinales Dihaloalkan eignet sich insbesondere Ethylendichlorid (EDC oder 1,2-Dichlorethan). Als Alkylenpoylamine werden insbesondere Ethylendiamin oder höhere Ethylenamine wie DETA, aber auch TETA und TEPA eingesetzt. Bei diesen Verfahren (EDC-Verfahren) fällt ein Gemisch verschiedener Ethylenamine (lineare Ethylenamine wie EDA, DETA, TETA, TEPA oder höhere Ethylenamine sowie cyclische Derivate wie Piperazin (Pip) oder Aminoethyl-Piperazin (AEPip)) an. Je nachdem welches Ethylenamin zu den Edukten EDC und NH₃ zugegeben wird, enthält das Reaktionsgemisch einen entsprechenden Anteil an höheren Ethylenaminen. Wenn beispielsweise gezielt TEPA hergestellt werden soll, wird den Edukten EDC und NH₃ das Ethylenamin TETA zugegeben. Auf diese Weise enthält das Produkt (Ethylenamingemisch) einen höheren Anteil an TEPA, jedoch auch die vorgenannten weiteren linearen sowie cyclischen Ethylenamine. Nachteilig an diesem Verfahren ist insbesondere, dass das Verfahren mit einer geringen Selektivität abläuft (Erhalt eines Ethylenamingemisches) und dass zuerst ein spezielles Ethylenamin hergestellt werden muss (beispielsweise DETA), welches anschließend in das Verfahren eingebracht wird, um das nächst höhere Ethylenamin (beispielsweise TETA) gezielt herzustellen bzw. die Ausbeute zu erhöhen. Dieses Verfahren stellt jedoch aufgrund der eingesetzten Edukte (Halogenalkane) und der anfallenden Salzsäure ein Korrosionsproblem sowie aufgrund der anfallenden Salze ein Umweltproblem dar.

US-A 3,462,493 betrifft ein Verfahren zur Herstellung TETA, wobei ein mindestens fünffach molarer Überschuss an EDA mit Ethylendichlorid oder Ethylendibromid umgesetzt wird. Als Nebenprodukte treten hierbei insbesondere Pip oder Piperazinoethylethylendiamin auf.

DE-T 689 11 508 beschreibt ein alternatives Verfahren zur Herstellung von linear verlängerter Polyalkylenpolyamine wie TETA. In diesem Verfahren wird ein bifunktioneller aliphatischer Alkohol mit einem Aminreaktanden in Anwesenheit eines Wolframhaltigen Katalysators umgesetzt. Als bifunktionaler aliphatischer Alkohol eignet sich insbesondere Monoethanolamin (MEA), als Aminreaktanden können beispielsweise EDA oder DETA eingesetzt werden. Durch dieses Verfahren werden prinzipiell Gemische aus linear verlängerten Polyalkylenpolyaminen (also Ethylenamingemische) erhalten. In diesen Ethylenamingemischen sind an Ethylenaminen DETA, TETA, TEPA, Pip, AEPip oder Piperazinderivate höherer Ethylenamine enthalten, wobei der Anteil der jeweiligen Komponente je nach eingesetzten Aminreaktanden variiert. Sofern als Aminreaktand DETA verwendet wird, wird ein Ethylenamingemisch mit hohem Anteil an TETA und TEPA erhalten. An diesem Verfahren ist nachteilig, dass das Verfahren mit einer geringen Selektivität abläuft (bezüglich der Komponenten des erhaltenen Ethylenamingemisches). und dass zuerst ein zusätzliches Ethylenamin synthetisiert werden muss, dass mit dem bifunktionalen aliphatischen Alkohol (beispielsweise MEA) umgesetzt wird. Hierbei entstehen in größerer Menge Nebenprodukte wie Aminoethylethanolamin (AEEA) oder höhere Hydroxy-haltige Ethylenamine, die von geringerem wirtschaftlichem Interesse sind. Die größere Menge an anfallenden Nebenprodukten ist darin begründet, da MEA bzw. die höheren Ethanolamine (z.B. AEEA) anstelle mit dem eingesetzten Amin mit sich selbst reagieren können. Aufgrund der (statistisch) vielen Reaktionsmöglichkeiten ist die Selektivität zum linearen TETA wegen der Koppelprodukte recht gering und nicht steuerbar. Die Synthese ist nur bei Teilumsatz möglich.

Einen Überblick über die Herstellung von Ethylenaminen liefert der SRI-Report "CEH Product Review Ethyleneamines"; SRI International, 2003; S. 53-54, in dem entsprechend der vorstehend beschriebenen Verfahren (mit den Edukten EDC oder MEA) insbesondere EDA oder DETA hergestellt werden. Dabei fallen höhere Ethylenamine wie TETA oder TEPA als Nebenprodukte an bzw. werden durch erneute Umsetzung der Edukte mit EDA oder DETA in höherer Ausbeute erhalten.

Im Stand der Technik wird somit nirgendwo beschrieben, dass auch Gemische von α-Aminonitrilen, die beispielsweise AAN, IDAN, EDDN oder sonstige höhere α-Aminonitrile enthalten, hydriert werden können. Die Verfahren gemäß dem Stand der Technik beschränken sich vielmehr auf die Hydrierung von einzelnen Substanzen.

Aufgabe der vorliegenden Erfindung ist es demnach, ein einfaches und kostengünstiges Verfahren zur Herstellung von Ethylenaminen wie EDA, DETA, TETA, TEPA oder Pip bereitzustellen. Dabei soll ein hoher Umsatz bei jeweiliger hoher Selektivität erzielt werden, wobei das Verhältnis der Ethylenamine variabel ist.

Die Aufgabe wird gelöst durch ein Verfahren zur Herstellung eines Ethylenamingemisches, wobei ein Aminonitrilgemisch enthaltend mindestens 2 α-Aminonitrile zu jeweils mindestens 5 Gew.-% in Gegenwart eines Katalysators und gegebenenfalls einem Lösungsmittel hydriert wird. Hydrieren bedeutet im Rahmen der vorliegenden Erfindung die Reaktion des Aminonitrilgemisches mit Wasserstoff.

Unter dem Begriff "α-Aminonitril" soll im Rahmen der vorliegenden Erfindung jede kohlenwasserstoffhaltige Verbindung verstanden werden, die mindestens eine Cyanomethylaminogruppe (-NH-CH₂-CN) enthält. Vorzugsweise enthalten die α-Aminonitrile zusätzlich mindestens eine Ethylengruppe, eine weitere Cyanogruppe und/oder mindestens eine weitere primäre, sekundäre oder tertiäre Aminogruppe. AAN ist ebenfalls ein bevorzugtes Aminonitril.

Bevorzugte α-Aminonitrile sind ausgewählt aus Aminoacetonitril (AAN), Iminodiacetonitril (IDAN), Ethylendiamindiacetonitril (EDDN), Ethylendiaminmonoacetonitril (EDMN), Diethylentriamindiacetonitril (DETDN), Diethylentriaminmonoacetonitril (DETMN), Piperazinylethylaminoacetonitril (PEAN), Aminoethylpiperazinylacetonitril (AEPAN) und Cyanomethylpiperazinylethylaminoacetonitril (CMPEAN). Weitere α-Aminonitrile, die im Aminonitrilgemisch enthalten sein können, sind z.B. Nitrilotrisacetonitril (NTAN) und Diethylentriamintriacetonitril (DETTN).

Zur Verdeutlichung sind für die vorstehend aufgeführten Aminonitrile nachfolgend deren chemische Strukturformeln angegeben:

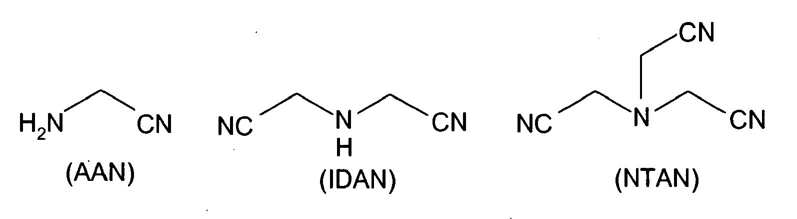

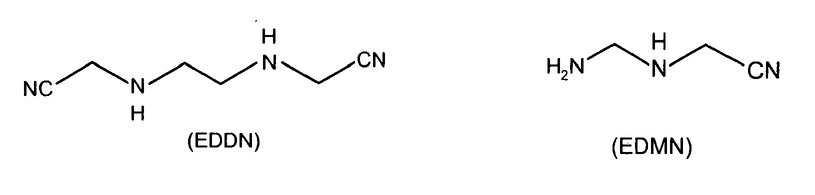

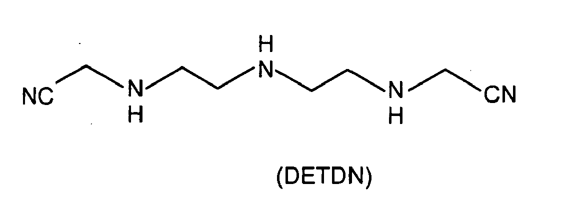

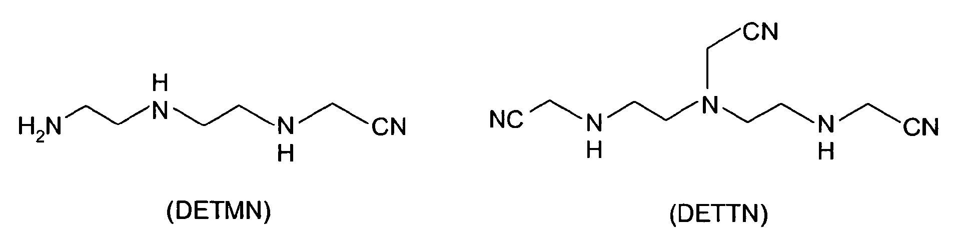

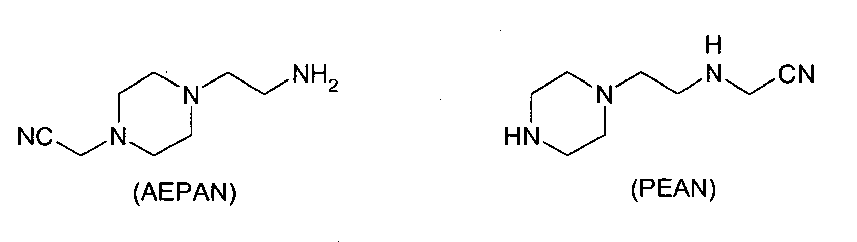

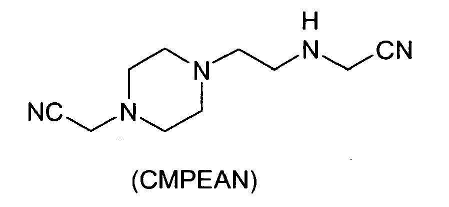

Besonders bevorzugte α-Aminonitrile sind ausgewählt aus AAN, IDAN, EDDN, EDMN, DETDN oder DETMN.

Unter dem Begriff "Ethylenamin" soll im Rahmen der vorliegenden Erfindung jede kohlenwasserstoffhaltige Verbindung verstanden werden, die mindestens ein Ethylendiamin-Fragment (-NH-CH₂-CH₂-NH-) enthält. Vorzugsweise enthalten die Ethylenamine zusätzlich mindestens eine weitere Ethyleneinheit und eine weitere primäre, sekundäre oder tertiäre Aminogruppe. EDA ist ebenfalls ein bevorzugtes Ethylenamin. Als Ethylenamin sollen im Rahmen der vorliegenden Erfindung auch cyclische Verbindungen wie beispielsweise Piperazin (Pip) sowie dessen Derivate verstanden werden.

Ethylenamine, die im erfindungsgemäßen Verfahren als Haupt- oder Nebenprodukt im Ethylenamingemisch enthalten sein können, sind ausgewählt aus

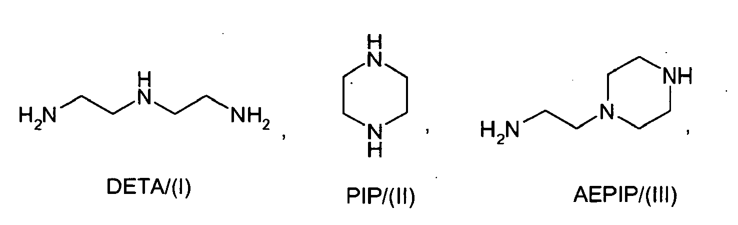

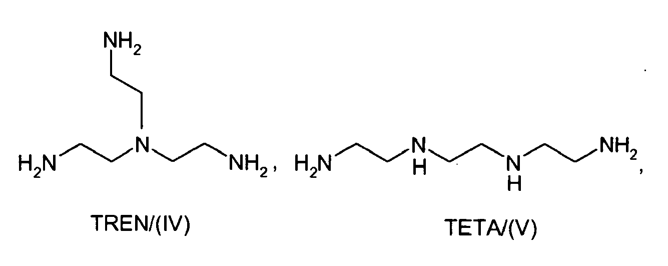

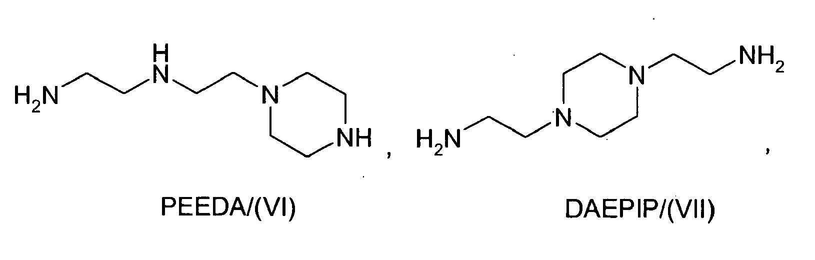

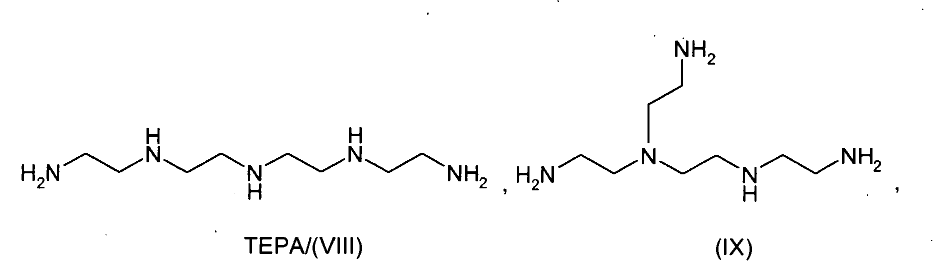

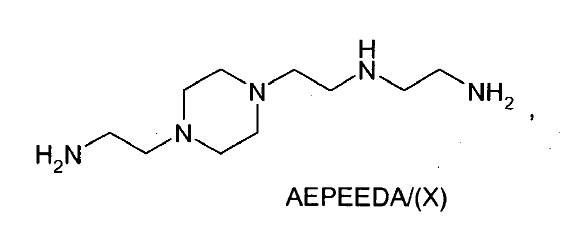

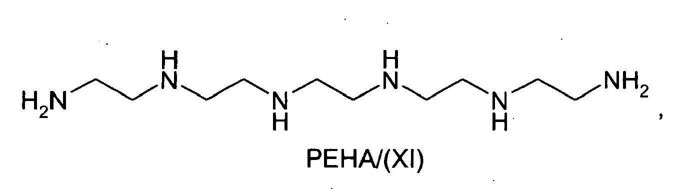

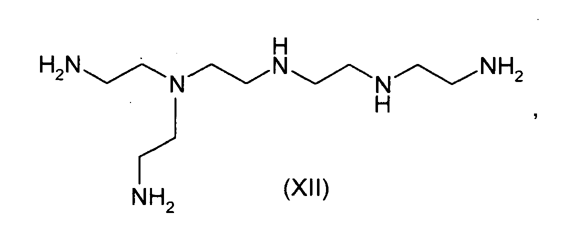

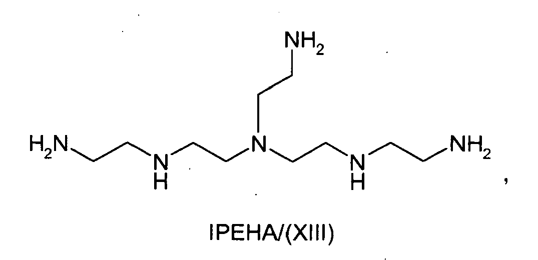

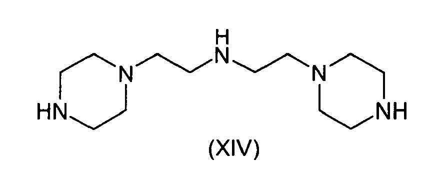

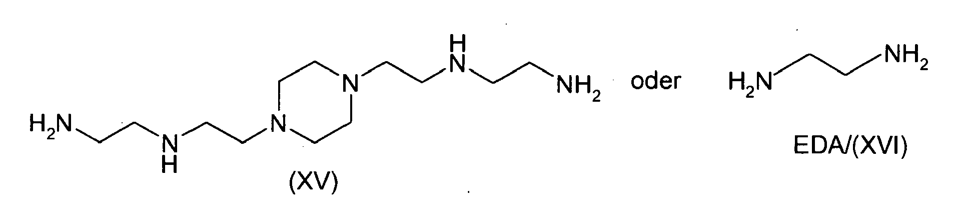

Bevorzugte Ethylenamine sind ausgewählt aus Ethylendiamin (EDA), Diethylentriamin (DETA), Triethylentetraamin (TETA), Tetraethylenpentaamin (TEPA), Pentaethylenhexaamin (PEHA), Piperazin (Pip) oder Aminoethylpiperazin (AEPip).

Besonders bevorzugte Ethylenamine sind EDA, DETA, TEPA oder TETA.

Der Vollständigkeit halber ist erwähnt, dass von den vorstehend abgebildeten Ethylenaminen die Verbindung (VI) als Piperazinethylethylendiamin (PEEDA), die Verbindung (VII) als Diaminoethylpiperazin (DAEPip), die Verbindung (X) als Aminoethylpiperazinethylethylendiamin (AEPEEDA) und die Verbindung (XIII) als Iso-Pentaethylenhexaamin (IPEHA) bezeichnet werden.

Das erfindungsgemäße Verfahren hat den Vorteil, dass lineare Ethylenamine, insbesondere EDA, DETA, TETA oder TEPA bei hohem Umsatz und/oder Selektivität hergestellt werden können. Die erhöhte Selektivität zeigt sich insbesondere darin, dass beispielsweise das eingesetzte AAN überwiegend zu EDA oder das eingesetzte EDDN überwiegend zu TETA hydriert wird. Im Vergleich zu den bekannten TETA- oder TEPA-Herstellungsverfahren kann somit das gewünschte Hauptprodukt (TETA oder TEPA) gezielt zudem in hoher Ausbeute und Selektivität hergestellt werden. Die dabei gebildeten Nebenprodukte können weitere lineare oder cyclische Ethylenamine sein. Der Anteil an cyclischen Ethylenaminen ist im erfindungsgemäßen Verfahren relativ gering. Diese Nebenprodukte sind jedoch teilweise ebenfalls interessante Wertprodukte, deren Isolierung beispielsweise in großtechnischen Verfahren lohnenswert ist. Durch geeignete Zusammenstellung an Aminkomponente und Formaldehydcyanhydrin bzw. Formaldehyd und Blausäure sowie Rückführungen einiger Aminkomponenten kann in dem erfindungsgemäßen Verfahren der Produktmix den Bedürfnissen des Marktes angepasst werden.

In vorteilhafter Weise werden die eingesetzten α-Aminonitrile wie AAN, IDAN oder EDDN vollständig oder nahezu vollständig umgesetzt. Dies ist insbesondere bei großtechnischen Verfahren von Bedeutung, da nicht umgesetztes Edukt in der Regel in den Produktionskreislauf zurückgeführt wird bzw. entsorgt werden muss. Verfahren, bei denen größere Mengen an α-Aminonitrilen nicht umgesetzt werden, sind aufgrund der hohen Instabilität der α-Aminonitrile von besonderem Nachteil. Einerseits neigen sowohl AAN, IDAN oder EDDN dazu, sich bei höheren Temperaturen zu zersetzen, so dass die Zersetzungsprodukte nicht in den jeweiligen Kreislauf zurückgeführt werden können, andererseits kann diese Zersetzung auch mit explosionsartiger Heftigkeit verlaufen. Auch kann die bei der Zersetzung freiwerdende Blausäure den Katalysatorverbrauch deutlich erhöhen. Da im erfindungsgemäßen Verfahren die Aminonitrile vollständig umgesetzt werden können, müssen hinsichtlich der Rückführung in den Produktionszyklus keine Anstrengungen unternommen werden.

Ein weiterer Vorteil des erfindungsgemäßen Verfahrens ist, dass im Gegensatz zum EDC-Verfahren auf den Einsatz von chlorierten Kohlenwasserstoffen als Edukt verzichtet werden kann. Zudem fallen keine Salzsäure bzw. deren Salze als weiteres Reaktionsprodukt an. Die Entsorgung der vorgenannten Stoffe ist insbesondere bei großtechnischen Verfahren ein (Umwelt-)Problem. Vorteilhaft im Vergleich zum MEA-Verfahren ist, dass aufgrund der unterschiedlichen Edukte die Bildung von AEEA sowie weiterer Verbindungen mit Hydroxy-Funktion keine Rolle spielt. Ebenso ist es ein Vorteil, dass das erfindungsgemäße Verfahren kontinuierlich durchgeführt werden kann.

Ein Vorteil, ein Ethylenamingemisch - anstelle der Herstellung der einzelnen Komponenten in separaten Kampagnen bzw. in getrennten Verfahren - herzustellen ist, dass auf die Zudosierung von Ammoniak in der Hydrierung verzichtet werden kann. Bei der gezielten Herstellung von Ethylenaminen gemäß Stand der Technik werden in der Regel Ammoniak oder andere Additive zur Unterdrückung von sekundären Aminen hinzugefügt. Bei der erfindungsgemäßen Synthese von Ethylenamingemischen ist die Unterdrückung der Dimerisierung nicht notwendig, da Dimere des Produktmixes Wertprodukte darstellen. So führt beispielsweise die Dimerisierung von EDA zu DETA oder EDA und TETA zu TEPA.

Trotz der Tatsache, dass im erfindungsgemäßen Verfahren prinzipiell ein Ethylenamingemisch anfällt, können durch kontinuierliche Isolierung die Hauptkomponenten wie EDA, DETA, TETA oder TEPA und gegebenenfalls die als Nebenprodukte anfallenden weiteren Ethylenamine erhalten werden. Bei herkömmlichen Verfahren, bei denen jeweils die Aminonitrile AAN oder IDAN separat hydriert werden, fallen prinzipiell immer DETA, EDA bzw. weitere Ethylenamine (jeweils abhängig vom verwendeten Edukt) als Nebenprodukte an. Demzufolge sind im Anschluss an die jeweiligen gezielten Ethylenamin-Synthesen generell die gleichen Aufreinigungsschritte wie im erfindungsgemäßen Verfahren zur Abtrennung der Nebenprodukte vom entsprechenden Hauptprodukt erforderlich. Verfahren zur Abtrennung der bei den Einzelverfahren jeweils anfallenden Nebenprodukte (beispielsweise DETA bzw. EDA) unterscheiden sich somit prinzipiell nicht von Verfahren zur Isolierung der im erfindungsgemäßen Verfahren anfallenden Hauptprodukte (z.B. EDA und DETA), lediglich die abzutrennende Menge an EDA bzw. DETA ist.verschieden. Bei Kampagnenfahrweise kommt zudem auch nur die diskontinuierliche Fahrweise in Frage, was aufgrund der gewünschten Mengen unpraktikabel ist. Bei kontinuierlicher Fahrweise sind Abstellungen und Umstellungen der Anlagen in Kauf zu nehmen (Reduzierung der Anlagenverfügbarkeit, Reinigungsaufwand, Produktverlust, Personeller Aufwand etc.). Auch müssen für die Bedürfnisse des Marktes entsprechende Lagerkapazitäten vorhanden sein.

Ebenso ist es als Vorteil anzusehen, dass, je nach Bedürfnissen des Marktes, ein höherer oder niedriger Anteil an EDA, DETA, TETA oder TEPA bzw. der weiteren Ethylenamine im Ethylenamingemisch hergestellt werden kann. In vorteilhafter Weise wird das erfindungsgemäße Verfahren kontinuierlich durchgeführt. Dies ist darin begründet, dass sich prinzipiell das Verhältnis von beispielsweise der Edukte IDAN zu AAN oder EDDN zu AAN im Produkt hinsichtlich DETA zu EDA oder TETA zu EDA wieder findet. So können im erfindungsgemäßen Verfahren spezielle Aminonitrilgemisch-Zusammensetzungen gezielt eingesetzt werden, um die im Markt gewünschten Mengenverhältnisse zu bedienen.

Die optimalen Fahrbedingungen können sich bei der Hydrierung von einzelnen Aminonitrilen deutlich unterscheiden. Bei der erfindungsgemäßen Hydrierung eines Aminonitrilgemisches sind die einzustellenden Fahrbedingungen je nach Zusammensetzung jedoch nur leicht unterschiedlich und dadurch leichter optimierbar. Somit ist eine Flexibilität der eingesetzten Maschinen und Apparate nur in geringem Maße erforderlich, wie sie standardmäßig von marktüblichen Geräten vorhanden ist (z.B. Förderleistung von Pumpen, Betriebstemperatur von Wärmeübertragern, Druckauslegung der Apparate etc.).

Ein weiterer Vorteil der erfindungsgemäßen Hydrierung von Aminonitrilgemischen, insbesondere solche, die AAN enthalten, gegenüber der separaten Herstellung von beispielsweise DETA oder gegebenenfalls der weiteren Ethylenaminen ist deren hohe Komplexierung des Katalysators. Die daraus resultierende Produkthemmung hat eine langsamere Hydriergeschwindigkeit zur Folge. Bei der Herstellung von EDA ist die Produkthemmung deutlich geringer, so dass eine höhere Hydriergeschwindigkeit von AAN, möglich ist. Sofern die Hydrierung von IDAN oder gegebenenfalls im weiteren Aminonitrile in Gegenwart von vorzugsweise mindestens 5 Gew.-% AAN wie im erfindungsgemäßen Verfahren durchgeführt wird, ist die Produkthemmung der entsprechenden Ethylenamine reduziert. Für ein gegebenes Aminonitrilgemisch ist somit die Raum-Zeit-Ausbeute der jeweiligen Komponenten größer als bei der entsprechenden Hydrierung der Einzelkomponenten, bzw. die Hydrierung des Gemisches kann bei deutlich niedrigeren Drücken durchgeführt werden.

Das erfindungsgemäße Verfahren geht von einem Aminonitrilgemisch als Edukt aus. Das Aminonitrilgemisch enthält mindestens 2 α-Aminonitrile, wobei mindestens zwei dieser α-Aminonitrile zu mindestens 5 Gew.-% in dem Aminonitrilgemisch enthalten sind. Im Rahmen des erfindungsgemäßen Verfahrens werden also auch Aminonitrilgemische verstanden, die beispielsweise fünf unterschiedliche α-Aminonitrile enthalten, wobei jedoch nur zwei dieser α-Aminonitrile zu mindestens 5 Gew.-% in dem Aminonitrilgemisch enthalten sind, während die übrigen drei α-Aminonitrile zu weniger als 5 Gew.-% enthalten sein können. Gegebenenfalls können zusätzlich auch weitere Aminonitrile, wie β-Aminonitrile, im Aminonitrilgemisch enthalten sein. Die vorstehenden sowie nachfolgenden Gewichtsprozentangaben bezüglich der einzelnen im Aminonitrilgemisch enthaltenen α-Aminonitrile beziehen sich auf die Gesamtmenge der im Gemisch enthaltenen α-Aminonitrile. Gegebenenfalls vorhandenes Lösungsmittel (Wasser oder organisches Lösungsmittel) oder weitere Additive sind bei diesen Mengenangaben nicht berücksichtigt. Sämtliche Mengenangaben sind so zu verstehen, dass die Summe der im Aminonitrilgemisch enthaltenen α-Aminonitrile 100 Gew.-% nicht übersteigt.

In einer bevorzugten Ausführungsform der vorliegenden Erfindung enthält das Aminonitrilgemisch mindestens 2 α-Aminonitrile, die jeweils zu mindestens 10 Gew.-% in dem Aminonitrilgemisch enthalten sind. In einer weiteren bevorzugten Ausführungsform sind in dem Aminonitrilgemisch mindestens zwei Aminonitrile zu mindestens 10 Gew.-% und mindestens ein weiteres, vorzugsweise zwei weitere Aminonitrile zu (jeweils) mindestens 5 Gew.-% enthalten. Weiterhin ist es bevorzugt, dass AAN zu mindestens 5 Gew.-%, vorzugsweise zu mindestens 10 Gew.-%, im Aminonitrilgemisch enthalten ist.

In einer Ausführungsform der vorliegenden Erfindung wird ein Aminonitrilgemisch hydriert enthaltend mindestens zwei der Komponenten a) bis d) mit
a) 10 bis 75 Gew.-% AAN,
b) 10 bis 50 Gew.-% IDAN, EDMN oder ein Gemisch davon,
c) 10 bis 70 Gew.-% EDDN, DETMN oder ein Gemisch davon,
d) 5 bis 50 Gew.-% DETDN, und
e) 0 bis 10 Gew.-% PEAN, AEPAN, CMPEAN oder ein Gemisch davon.

In weiteren Ausführungsformen der vorliegenden Erfindung werden Aminonitrilgemische hydriert, die mindestens 5 Gew.-%, vorzugsweise mindestens 30 Gew.-% AAN und mindestens 5 Gew.-%, vorzugsweise 5 bis 50 Gew.-% IDAN enthalten.

In weiteren Ausführungsformen der vorliegenden Erfindung werden Aminonitrilgemische hydriert, die mindestens 5 Gew.-%, vorzugsweise mindestens 30 Gew.-% EDDN und mindestens 5 Gew.-%, vorzugsweise 5 bis 50 Gew.-% EDMN enthalten.

In weiteren Ausführungsformen der vorliegenden Erfindung werden Aminonitrilgemische hydriert, die mindestens 5 Gew.-%, vorzugsweise mindestens 30 Gew.-% DETDN und mindestens 5 Gew.-%, vorzugsweise 5 bis 50 Gew.-% DETMN enthalten.

Generell kann jede Art/Qualität der vorstehend beschriebenen α-Aminonitrile wie beispielsweise AAN, IDAN, EDMN, EDDN, DETMN oder DETDN eingesetzt werden. Gegebenenfalls können die entsprechenden Aminonitrile auch in Form ihrer wässrigen oder wässrigen ammoniakalischen Lösungen eingesetzt werden. Verfahren zur Herstellung von beispielsweise AAN oder IDAN sind dem Fachmann bekannt. Vorzugsweise werden AAN und/oder IDAN durch Umsetzung von NH₃ und Formaldehydcyanhydrin (FACH) hergestellt.

Ebenso sind dem Fachmann Verfahren zur Herstellung von EDDN oder EDMN bekannt. Siehe hierzu K. Masuzawa et al., Bull. Chem. Soc. Japan, Band 41 (1968), Seiten 702-707; H. Brown et al., Helvetica Chimica Acta, Band 43 (1960), Seiten 659-666 und H. Baganz et al., Chem. Ber., 90 (1957), Seiten 2944-2949. Vorzugsweise werden EDDN und/oder EDMN durch Umsetzung von EDA und FACH hergestellt. Vorzugsweise ist das molare Verhältnis von EDA zu FACH 1 : 1,5 bis 1 : 2 [mol/mol]. Ein neues Verfahren zur Herstellung von EDDN oder Gemischen enthaltend EDDN und EDMN ist von der Anmelderin der vorliegenden Erfindung zeitgleich angemeldet worden.

DETDN und DETMN sind neue Verbindungen, die bis jetzt noch nicht in der Literatur beschrieben worden sind. Ein Verfahren zur Herstellung von DETDN beziehungsweise Gemische enthaltend DETDN und DETMN ist von der Anmelderin der vorliegenden Erfindung zeitgleich angemeldet worden.

DETDN wird vorzugsweise hergestellt durch Umsetzung von DETA und FACH. Vorzugsweise beträgt das molare Verhältnis von DETA zu FACH 1 : 1,5 bis 1 : 2 [mol/mol]. Mehr bevorzugt beträgt das molare von DETA zu FACH 1 : 1,8 bis 1 : 2 [mol/mol]. Verfahren zur Herstellung von DETA und FACH sind dem Fachmann bekannt. Vorzugsweise wird DETA im erfindungsgemäßen Verfahren in Form seiner freien Base eingesetzt, gegebenenfalls können jedoch auch Salze wie das Dihydrochlorid von DETA als Edukt verwendet werden. Vorzugsweise erfolgt die Herstellung von DETDN in einem Lösungsmittel, insbesondere in Gegenwart von Wasser. DETMN kann hergestellt werden durch entsprechende Erniedrigung des molaren Anteils an FACH bezüglich DETA im Vergleich zur DETDN-Herstellung.

PEAN, AEPAN und CMPEAN sind ebenfalls neue Verbindungen, die bis jetzt noch nicht in der Literatur beschrieben worden sind. Diese 3 cyclischen Aminonitrile fallen einzeln oder als Gemisch bei der Umsetzung von AEPip mit FACH an. Die Umsetzungsbedingungen von AEPip mit FACH entsprechen prinzipiell den vorstehend aufgeführten Umsetzungsbedingungen für DETA mit FACH. Vorzugsweise beträgt das molare Verhältnis von AEPip zu FACH 1 : 1,5 bis 1 : 2 [mol/mol].

Prinzipiell können die einzelnen im Aminonitrilgemisch enthaltenen α-Aminonitrile getrennt voneinander synthetisiert werden und vor dem Einsatz im erfindungsgemäßen Verfahren in den entsprechenden Mengen zum Aminonitrilgemisch vereint werden. Gegebenenfalls können einzelne bzw. alle α-Aminonitrile auch gemeinsam synthetisiert werden, beispielsweise AAN und IDAN, EDDN und EDMN oder DETDN und DETMN. Die im Aminonitrilgemisch erforderlichen Mengenkonzentrationen durch gezielten Einsatz der Aminkomponenten können gegebenenfalls durch Zugabe der entsprechenden α-Aminonitrile durch entsprechendes Auf- und/oder Abkonzentrieren hergestellt werden.

In einer Ausführungsform der vorliegenden Erfindung werden die Hydrierungsprodukte DETA, EDA und/oder AEPip ganz oder teilweise zurückgeführt. Die zurückgeführten Hydrierungsprodukte DETA, EDA und/oder AEPip werden zusammen oder separat mit FACH umgesetzt, wobei vorzugsweise die α-Aminonitrile DETDN und/oder DETMN (Rückführung von DETA), EDDN und/oder EDMN (Rückführung von EDA) oder PEAN, AEPAN und/oder CMPEAN (Rückführung von AEPip) erhalten werden. Die auf diese Weise hergestellten α-Aminonitrile werden wiederum der Hydrierung zugeführt. Je nachdem, welche und in welchem Umfang die Hydrierungsprodukte DETA, EDA und/oder AEPip in den Kreislauf zurückgeführt werden, kann der jeweilige Anteil der einzelnen Komponenten des Ethylenamingemisches gesteuert werden.

In einer weiteren Ausführungsform der vorliegenden Erfindung werden vor der Hydrierung die Leichtsieder aus dem Aminonitrilgemisch abgetrennt. Sofern zur Herstellung der α-Aminonitrile FACH verwendet wird, kann die Leichtsiederabtrennung bereits vor Umsetzung des FACHs mit NH₃, EDA, DETA oder AEPip erfolgen.

Vorzugsweise werden als Leichtsieder Blausäure (HCN) abgetrennt. Vorzugsweise erfolgt die Abtrennung durch Destillation, beispielsweise in Form einer Dünnschichtdestillation wie z. B. einer Sambay Destillation ("Chemie Ingenieur Technik, Vol. 27, S. 257-261"). Gegebenenfalls kann das Reaktionsgemisch auch mit Stickstoff gestrippt werden. Weiterhin kann vor der Hydrierung des Aminonitrilgemisches auch eine Abreicherung von Wasser, vorzugsweise durch Destillation, erfolgen. Ebenfalls ist eine Absorption von Verunreinigungen an einem Absorbens, zum Beispiel Aktivkohle oder Ionentauscher, möglich.

In denjenigen Ausführungsformen des erfindungsgemäßen Verfahrens, in denen das Aminonitrilgemisch weniger als 5 Gew.-% AAN enthält, erfolgt die Hydrierung in Gegenwart eines Lösungsmittels, beispielsweise einem organischen Lösungsmittel und/oder Wasser. Die (zusätzliche) Verwendung eines organischen Lösungsmittels (inerten organischen Verbindung) und/oder von Wasser erweist sich jedoch als vorteilhaft, da insbesondere durch die Verwendung von einem organischen Lösungsmittel eine Stabilisierung der einzelnen Komponenten des Aminonitrilgemisches, insbesondere in Gegenwart der resultierenden Amine, erzielt werden kann. Außerdem lässt sich durch die Verwendung von Lösungsmitteln ein Spüleffekt an dem eingesetzten Katalysator erzielen, wodurch dessen Standzeit erhöht bzw. dessen Verbrauch erniedrigt (längere Katalysatorlebensdauer) sowie die Katalysatorbelastung verbessert werden kann.

Ein geeignetes Lösungsmittel, welches ein oder mehrere Komponenten umfassen kann, sollte bevorzugt folgende Eigenschaften aufweisen:
(a) das Lösungsmittel sollte stabilisierend auf die Komponenten des Aminonitrilgemisches wirken, insbesondere eine Zersetzung von AAN oder IDAN bei den herrschenden Temperaturen reduzieren;
(b) das Lösungsmittel sollte eine gute Wasserstofflöslichkeit zeigen;
(c) das Lösungsmittel sollte bei den Reaktionsbedingungen inert sein;
(d) das Reaktionsgemisch (Aminonitrilgemisch sowie Lösungsmittel) sollte unter Reaktionsbedingungen einphasig sein;
(e) die Lösemittelauswahl sollte im Hinblick auf eine bevorzugt destillative Abtrennung des Produktes im Anschluss an die Hydrierung aus dem Produktstrom erfolgen, wobei energie- oder apparativ-aufwendige (z.B. engsiedende Gemische oder schwierig zu trennende Azeotrope) Trennung zu vermeiden sind.
(f) das Lösungsmittel sollte gut von den Produkten abtrennbar sein, d.h. die Siedetemperatur sollte sich hinreichend von der der Produkte unterscheiden. Hierbei wird eine niedrigere Siedetemperatur als die der Produkte bevorzugt.

Mögliche Lösungsmittel sind organische Lösungsmittel, beispielsweise Amide, wie N-Methylpyrrolidon (NMP) und Dimethylformamid (DMF), aromatische und aliphatische Kohlenwasserstoffe, wie Benzol und Xylol, Alkohole wie Methanol, Ethanol, Propanol, Isopropanol, Butanol, Isobutanol, sekundäres Butanol und tertiäres Butanol, Amine, wie Ethylenamine, Alkylamine, Ammoniak, Ester, wie Essigsäuremethylester oder Essigsäureethylester und Ether, wie Diisopropylether, Diisobutylether, Glykoldimethylether, Diglykoldimethylether, Dioxan und Tetrahydrofuran (THF). Bevorzugt werden im erfindungsgemäßen Verfahren Ether verwendet, mehr bevorzugt cyclische Ether und besonders bevorzugt Tetrahydrofuran. In einer weiteren bevorzugten Ausführungsform werden Alkohole, insbesondere Methanol, als organisches Lösungsmittel verwendet.

Das Lösungsmittel wird im Gewichtsverhältnis zu dem eingesetzten Aminonitrilgemisch von 0,1 : 1 bis 15 : 1 eingesetzt. Die Konzentration des Aminonitrilgemisches in der Lösung, in der die Hydrierung durchgeführt wird, sollte so gewählt werden, dass eine geeignete Zuführrate bzw. Verweilzeit eingestellt werden kann. Es ist bevorzugt, das Aminonitrilgemisch zu 10 bis 50 Gew.-% mit dem Lösungsmittel zu vermischen. Bezogen auf die besonders bevorzugten Lösungsmittel Methanol bzw. Tetrahydrofuran ist es beispielsweise vorteilhaft, das Aminonitrilgemisch zu 20 bis 40 Gew.-% bezogen auf das Lösungsmittel einzusetzen.

Sofern Wasser vorhanden ist, liegt der Anteil an Wasser in der Lösung in einem Bereich von 0 bis 70 Gew.-%, vorzugsweise bei 10 bis 50 Gew.-%. Die Mengenangaben des Wassers beziehen sich dabei auf das Aminonitril-Wassergemisch.

Gegebenenfalls können in der Lösung, in der die Hydrierung durchgeführt wird, zusätzliche Additive enthalten sein. Als Additive kommen prinzipiell Hydroxide wie Alkalimetallhydroxide, Alkoholate, Amide, Amine sowie gegebenenfalls Ammoniak in Frage. Vorzugsweise eignen sich als Additive Amine, wie z.B. EDA und Ammoniak, insbesondere EDA. Weiterhin können auch saure Additive, wie zum Beispiel Silikate zusätzlich in der Lösung enthalten sein. Diese Substanzen können als Reinstoff oder gelöst in einem Lösungsmittel zugesetzt werden. Vorzugsweise wird das erfindungsgemäße Verfahren unter Zusatz von Additiven durchgeführt, sofern mindestens 5 Gew.-% von EDDN, EDMN, DETDN oder DETMN im Aminonitrilgemisch enthalten sind.

In einer bevorzugten Ausführungsform der vorliegenden Erfindung wird die Hydrierung des Aminonitrilgemisches in Gegenwart von EDA durchgeführt. Um eine hohe Selektivität und/oder Umsatz an beispielsweise höheren, insbesondere linearen Ethylenaminen (wie TETA und/oder TEPA) zu erzielen, wird die Hydrierung des entsprechenden Aminonitrilgemisches in Gegenwart von EDA durchgeführt. Die Zugabe weiterer Additive ist nicht erforderlich, sie können jedoch ebenfalls zugegeben werden. Sofern das entsprechende Aminonitrilgemisch AAN enthält, ist aufgrund des bei der Hydrierung entstehenden EDA keine extra Zugabe oder Rückführung von EDA erforderlich.

In einer weiteren Ausführungsform liegt bei der Hydrierung eines AAN enthaltenden Aminonitrilgemisches aus der AAN-Synthese stammender, in Wasser gelöster Ammoniak vor. Dieser Überschuss an Ammoniak kann mit in die Hydrierung überführt werden und mit eventuell durch Kondensationsreaktionen anfallendem Ammoniak im Anschluss an die Hydrierung abgetrennt werden. Diese geringen Mengen haben keinen deutlichen Einfluss auf den Eigendruck im System.

Sofern noch Ammoniak in den Edukten bzw. in der gegebenenfalls eingesetzten wässrigen Lösung gelöst ist bzw. als Nebenprodukt bei der Hydrierung freigesetzt wird, ist dies nicht störend. Gegebenenfalls vorhandener Ammoniak kann nach dem Fachmann bekannten Methoden, beispielsweise destillativ, entfernt werden.

Als Katalysatoren zur Hydrierung der Nitril-Funktion zum Amin können Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten. In einer bevorzugten Ausführungsform werden im erfindungsgemäßen Verfahren Raney-Katalysatoren eingesetzt, bevorzugt Raney-Kobalt- oder Raney-Nickel-Katalysatoren und besonders bevorzugt mit mindestens einem der Elemente Cr, Ni oder Fe dotierte Raney-Kobalt- oder mit einem der Elemente Mo, Cr oder Fe dotierte Raney-Nickel-Katalysatoren.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff enthaltenden Gasstrom bei erhöhter Temperatur aktiviert. Wenn die Katalysatoren außerhalb des Reaktors reduziert werden, kann danach eine Passivierung durch einen Sauerstoff enthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können.

Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31,5 Gew.-% ZrO₂, 50 Gew.-% NiO, 17 Gew.-% CuO und 1,5 Gew.-% MoO₃. Ebenso geeignet sind die in WO-A 99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als 'Metallschwamm' aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach z. B. mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typischerweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

Bevorzugt wird erfindungsgemäß ein Kobalt-Skelett-Katalysator eingesetzt, der aus einer Co/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni, Cr enthält.

Solche Katalysatoren enthalten typischerweise neben Kobalt noch 1 bis 30 Gew.-% Al, besonders 2 bis 12 Gew.-% Al, ganz besonders 3 bis 6 Gew.-% Al, 0 bis 10 Gew.-% Cr, besonders 0,1 bis 7 Gew.-% Cr, ganz besonders 0,5 bis 5 Gew.-% Cr, insbesondere 1,5 bis 3,5 Gew.-% Cr, 0 bis 10 Gew.-% Fe, besonders 0,1 bis 3 Gew.-% Fe, ganz besonders 0,2 bis 1 Gew.-% Fe, und/oder 0 bis 10 Gew.-% Ni, besonders 0,1 bis 7 Gew.-% Ni, ganz besonders 0,5 bis 5 Gew.-% Ni, insbesondere 1 bis 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:
Al: 2 bis 6 Gew.-%, Co: ≥ 86 Gew.-%, Fe: 0 bis 1 Gew.-%, Ni: 1 bis 4 Gew.-%, Cr: 1,5 bis 3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Cr enthält.

Solche Katalysatoren enthalten typischerweise neben Nickel noch
1 bis 30 Gew.-% Al, besonders 2 bis 20 Gew.-% Al, ganz besonders 5 bis 14 Gew.-% Al,
0 bis 10 Gew.-% Cr, besonders 0,1 bis 7 Gew.-% Cr, ganz besonders 1 bis 4 Gew.-% Cr,
und/oder
0 bis 10 Gew.-% Fe, besonders 0,1 bis- 7 Gew.-% Fe, ganz besonders 1 bis 4 Gew.-% Fe,
wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden.

Dieser Katalysator weist folgende Zusammensetzung auf
Al: ≤14 Gew.-%, Ni: ≥ 80 Gew.-%, Fe: 1 bis 4 Gew.-%, Cr: 1 bis 4 Gew.-%.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

Die Temperaturen, bei denen das erfindungsgemäße Verfahren durchgeführt wird, liegen in einem Bereich von 40 bis 150°C, bevorzugt von 80 bis 140°C.

Der bei der Hydrierung herrschende Druck liegt im Allgemeinen bei 5 bis 300 bar, bevorzugt bei 30 bis 250 bar, besonders bevorzugt bei 70 bis 160 bar.

In einer bevorzugten Ausführungsform wird das Aminonitrilgemisch mit einer Rate der Hydrierung zugeführt, die nicht größer ist als die Rate, mit der das Aminonitrilgemisch mit Wasserstoff bei der Hydrierung reagiert.

Die Zuführrate ist somit bevorzugt so einzustellen, dass quasi Vollumsatz erreicht wird. Dieses wird durch Temperatur, Druck, Art des Gemisches, Menge und Art des Katalysators, des Reaktionsmediums, Durchmischungsgüte des Reaktorinhalts, Verweilzeit etc. beeinflusst.

Sofern im erfindungsgemäßen Verfahren ein Lösungsmittel verwendet wird, kann das Lösungsmittel zunächst vollständig mit dem Aminonitrilgemisch vermischt werden. Die erhaltene Lösung, die gegebenenfalls auch Additive enthalten kann, wird anschließend in das den Katalysator enthaltende Reaktionsgefäß zugeführt. Bei kontinuierlichen Verfahren kann eine Teilmenge des Lösungsmittels auch separat von der Lösung, die das Aminonitrilgemisch und das Lösungsmittel enthält, in das Reaktionsgefäß zugegeben werden. In der Ausführungsform des erfindungsgemäßen Verfahrens, in der ein Gemisch aus AAN und mindestens einem höheren Aminonitril eingesetzt wird, ist auch eine komplett separate Zudosierung des Lösemittels denkbar. In einer bevorzugten Ausführungsform wird das Aminonitrilgemisch als wässrige Lösung und das organische Lösemittel separat zudosiert.

In einer bevorzugten Ausführungsform erfolgt die Zuführung des in der Lösung enthaltenen Aminonitrilgemisches mit einer Rate, die nicht größer ist als die Rate, mit der das Aminonitrilgemisch mit Wasserstoff bei der Hydrierung reagiert.

Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird.

Das erfindungsgemäße Verfahren zur Herstellung von Ethylenaminen durch Hydrierung von Aminonitrilgemischen kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des Aminonitrilgemisches und des Katalysators mit dem gasförmigen Wasserstoff unter Druck möglich ist.

Die Hydrierung in Suspensionsfahrweise kann in einem Rührreaktor, Strahlschlaufenreaktor, Strahldüsenreaktor, Blasensäulenreaktor, bzw. in einer Kaskade derartiger gleicher oder verschiedener Reaktoren durchgeführt werden. Für die Hydrierung an einem Festbettkatalysator sind Rohreaktoren aber auch Rohrbündelreaktoren denkbar.

Im Fall eines Festbettkatalysator wird in Sumpf- oder Rieselfahrweise mit dem Aminonitrilgemisch beaufschlagt. Bevorzugt wird allerdings die Suspensionsfahrweise in semikontinuierlicher und bevorzugt in kontinuierlicher Fahrweise eingesetzt.

Die Hydrierung der Nitrilgruppen findet unter Freisetzung von Wärme statt, die in der Regel abgeführt werden muss. Die Wärmeabfuhr kann durch eingebaute Wärmeübertragerflächen, Kühlmantel oder außenliegende Wärmeübertrager in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen. Insbesondere kann der Kreislaufstrom mittels eines externen Wärmeübertragers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Das erfindungsgemäße Verfahren liefert ein Ethylenamingemisch, das als Hauptkomponente mindestens zwei Ethylenamine enthält, vorzugsweise mindestens zwei lineare Ethylenamine. Die Zusammensetzung des jeweiligen Ethylenamingemisches hängt stark von den verwendeten Edukten (α-Aminonitrile) ab. Wird beispielsweise ein Aminonitrilgemisch hydriert enthaltend als Hauptkomponenten AAN und IDAN, so findet sich prinzipiell das Verhältnis der Edukte nach der Hydrierung bei den entsprechenden Produkten EDA und DETA wieder. Je nach Hydrierbedingungen kann jedoch aus AAN weiteres DETA gebildet werden. Dadurch kann der DETA-Anteil des resultierenden Amingemisches um 1 bis 10 Gew.-% steigen. Sinngemäßes gilt auch für Gemische, die mehr als 2 α-Aminonitrile beziehungsweise weitere α-Aminonitrile enthalten.

Vorzugsweise sind nach der Hydrierung im Ethylenamingemisch mindestens ein, insbesondere mindestens zwei Ethylenamine enthalten ausgewählt aus Ethylendiamin (EDA), Diethylentriamin (DETA), Triethylentetraamin (TETA), Tetraethylenpentaamin (TEPA), Pentaethylenhexaamin (PEHA), Piperazin (Pip), Aminoethylpiperazin (AEPip), Piperazinethylethylendiamin (PEEDA) oder Diaminoethylpiperazin (DAEPip).

Im Anschluss an die Hydrierung kann das erhaltene Produkt (Ethylenamingemisch) gegebenenfalls weiter aufgereinigt werden, beispielsweise indem das gegebenenfalls verwendete Lösungsmittel und/oder der Katalysator nach dem Fachmann bekannten Methoden abgetrennt werden. Insbesondere können die Hauptprodukte (beispielsweise EDA, DETA, TETA oder TEPA) gemeinsam oder einzeln nach dem Fachmann bekannten Methoden aus dem Ethylenamingemisch isoliert werden. Sofern die Hauptprodukte gemeinsam isoliert werden, beispielsweise durch eine Destillation, können sie anschließend in die beiden Einzelprodukte isoliert werden. Letztendlich erhält man somit reines EDA, reines DETA, reines TETA sowie reines TEPA. Sonstige Verunreinigungen oder Nebenprodukte wie cyclische Ethylenamine (zum Beispiel Pip) können ebenfalls mit dem Fachmann bekannten Methoden aus dem Ethylenamingemisch abgetrennt werden. Gegebenenfalls. kann auch TETA gemeinsam mit den Piperazin-Derivaten DAEPip) und/oder PEEDA als "technisches TETA" isoliert werden.

Wie vorstehend bereits aufgeführt, können in einer Ausführungsform der vorliegenden Erfindung Aminonitrilgemische hydriert werden enthaltend mindestens zwei der Komponenten a) bis e) mit den entsprechenden Konzentrationsangaben.

In einer bevorzugten Ausführungsform werden Aminonitrilgemische hydriert enthaltend die Komponenten a) bis e) mit
a) zu 30 bis 70 Gew.-%, b) zu 15 bis 50 Gew.-%, c) zu 5 bis 25 Gew.-% und d) zu 5 bis 25 Gew.-%, e) 0 bis 5 Gew.-%,

Die genaue Zusammensetzung der Komponenten a) bis e) wird durch die Marktbedürfnisse geprägt. Hierbei wird EDA als Hauptkomponente neben höheren Ethylenaminen erhalten.

In einer weiteren bevorzugten Ausführungsform werden TETA und TEPA jeweils als Hauptkomponenten erhalten. Hier werden EDA und DETA parallel lediglich als notwendige Bausteine für eine eventuelle Rückleitung hergestellt. Somit ergeben sich hier Grenzen von
a) zu 5 bis 25 Gew.-%, b) zu 10 bis 30 Gew.-%, c) zu 25 bis 70Gew.-% und d) zu 5 bis 70 Gew.-%, e) 0 bis 5 Gew.-%.

Abhängig vom Markt sind aber auch weitere Grenzen denkbar.

In einer bevorzugten Ausführungsform wird das erfindungsgemäße Verfahren unter Verwendung von Tetrahydrofuran oder Methanol als Lösungsmittel durchgeführt. Die Temperatur bei der Hydrierung beträgt vorzugsweise 80 bis 140°C, der Druck vorzugsweise 30 bis 250 bar. Vorzugsweise wird der Hydrierung keine zusätzliche Ammoniakmenge zugeführt.

Die nachfolgenden Beispiele verdeutlichen das erfindungsgemäße Verfahren. Die Anteile sind in Gew.-% angegeben, sofern nicht anders aufgeführt. Ein mitgeführter interner Standard, Diethylenglykoldimethylether (DEGDME), erlaubt eine Quantifizierung des Produktes durch Bestimmung der eventuell gebildeten flüchtigen Zersetzungsbestandteile. Die Quantifizierung erfolgt mittels Gaschromatographie (GC), wobei den jeweils entnommenen Proben zur Homogenisierung Methanol zugegeben wird.

### Beispiele

### Formaldehydcyanhydrin:

In einem 6I-Reaktionsgefäß mit Propellerrührer werden 7000 g (70 mol) Formaldehyd (30 %) vorgelegt und mit Natronlauge (1 mol/l) ein pH-Wert von 5.5 eingestellt. Innerhalb 3 Stunden werden 1938 g (71,4 mol) Blausäure über ein auf 50 °C beheiztes U-Rohr unterhalb des Rührers gasförmig eindosiert, wobei die Reaktionstemperatur bei 30 °C und der pH-Wert bei 5.5 gehalten wird. Nach 10 Minuten Nachrührzeit wird der pH-Wert mit Schwefelsäure (50 %ig) auf 2.5 gestellt. Um Leichtsieder, insbesondere Blausäure abzutrennen, wird der Reaktionsaustrag einer Sambaydestillation (wie in "Chemie Ingenieur Technik, Vol. 27, S. 257-261 beschrieben) (1 mbar, 30°C) unterzogen. Über Liebig-Titration wird der entsprechende Gehalt ermittelt und durch Zugabe von Wasser ein Gehalt von 43,6 % FACH eingestellt.

### Beispiel 1

In einer integrierten Laboranlage bestehend aus einem Rohrreaktor mit vorgeschaltetem Mischer und anschließender Hydrierung im Autoklaven wird ein Aminonitrilgemisch ausgehend von FACH, Ammoniak und EDA bei 20 bar und 70 °C hergestellt, das anschließend zu den entsprechenden Ethylenaminen bei 50 bar und 120 °C hydriert wird.

Es werden kontinuierlich 137,3 g/h (1,05 mol/h) FACH, 47,4 g/h (2,8 mol/h) Ammoniak und 20,9 g/h (0,35 mol/h) EDA in den Rohrreaktor gefahren, was einem Verhältnis von FACH:NH₃:EDA von 3:5:1 entspricht. Der Reaktionsaustrag enthält 9,5 Gew.-% AAN und 21 Gew.-% EDDN, was einer Ausbeute von 33 % AAN und von 60 % EDDN bezogen auf eingesetztes FACH entspricht. Die Gesamtausbeute an Aminonitrilen beträgt 93 % mit einer Gesamtaminonitrilselektivität von 97 %.

Der Reaktionsaustrag wird ohne Entspannung bei 20bar mit einem internen Standard Diethylenglycoldimethylether (DEGDME) gemischt. 45g dieser Mischung wird in Gegenwart von 80g THF und 20NL Wasserstoff werden kontinuierlich pro Stunde in einem 270mL Autoklav mit Stromstörern und Scheibenrührer bei 50bar in Gegenwart von 10g Cr-dotierter Raney-Cobalt bei 120°C hydriert. Der Hydrieraustrag wird mittels GC analysiert. Aminonitrile können nicht mehr nachgewiesen werden. Neben 22 Gew.-% EDA werden, 0,7 Gew.-% Pip, 2,8 Gew.-% DETA sowie 16 Gew.-% AEPip und 41% TETA gefunden.

### Beispiel 2:

In einer integrierten Laboranlage bestehend aus einem Rohrreaktor mit vorgeschaltetem Mischer und anschließender Hydrierung im Autoklaven wird ein Aminonitrilgemisch ausgehend von FACH, Ammoniak und EDA bei 20 bar und 70 °C hergestellt, das anschließend zu den entsprechenden Ethylenaminen bei 50 bar und 120 °C hydriert wird. Es werden kontinuierlich 104,4 g/h (0,8 mol/h) FACH, 22,6 g/h (1,3 mol/h) Ammoniak und 15,9 g/h (0,26 mol/h) EDA in zwei hintereinander geschaltete Rohrreaktoren gefahren, was einem Verhältnis von FACH:NH₃:EDA von 3:5:1 entspricht. Der Reaktionsaustrag enthält 9,3 Gew.-% AAN, 0,3 Gew.-% IDAN und 21,5 Gew.-% EDDN, was einer Ausbeute von 30 % AAN, von 56 % EDDN und 1,2 % IDAN bezogen auf eingesetztes FACH entspricht. Die Gesamtausbeute an Aminonitrilen beträgt 87 % mit einer Gesamtaminonitrilselektivität von 87 %.

Der Reaktionsaustrag wird ohne Entspannung bei 20bar mit einem internen Standard Diethylenglycoldimethylether (DEGDME) gemischt. 45g dieser Mischung wird in Gegenwart von 80g THF und 20NL Wasserstoff werden kontinuierlich pro Stunde in einem 270mL Autoklav mit Stromstörern und Scheibenrührer bei 50bar in Gegenwart von 10g Cr-dotierter Raney-Cobalt bei 120°C hydriert. Der Hydrieraustrag wird mittels GC analysiert. Aminonirtrile können nicht mehr nachgewiesen werden. Neben 24 Gew% EDA werden 2 Gew.-% Pip, 7 Gew.-% DETA sowie 15 Gew.-% AEPip und 34% TETA gefunden.

### Beispiel 3 (kontinuierliche Hydrierung / 30 Gew.-% Wasser)

In einen 270ml-Autoklav mit Stromstörern und Scheibenrührer werden10g Cr-dotierter Raney-Kobalt vorgelegt und kontinuierlich 50 NL(Normliter)/h Wasserstoff zugefahren. Eine Mischung aus 30g AAN, 9g Wasser in 255g THF wird kontinuierlich pro Stunde bei 50 bar zugepumpt. Über eine Tauchfritte wird kontinuierlich Reaktionsgemisch ausgetragen. Die Reaktionstemperatur wird auf 120°C gehalten. Der Austrag wird über ein Regelventil entspannt. Regelmäßige Proben werden mittels GC analysiert. Zu keiner Zeit kann AAN im Austrag nachgewiesen werden. Die Proben zeigen gleich bleibend eine Selektivität von > 98 % EDA sowie 1% DETA.

Anschließend werden für 7h pro Stunde 24 g AAN, 10 g IDAN, 10 g Wasser sowie 255 g THF zugepumpt. In den GC-Analysen kann kein Nitril mehr nachgewiesen werden. Hierbei werden Selektivitäten von 66 % EDA, 30 % DETA sowie 1 % Piperazin erzielt.

Für weitere 7h werden pro Stunde neben 18g AAN (0,32mol) zusätzlich 22,5g IDAN in 255g THF inkl. 24g Wasser zudosiert. Auch in diesem Fall liegt Vollumsatz von AAN und IDAN vor. Die Selektivitäten des Gemischs liegen bei 41 % EDA, 51 % DETA sowie 3% Piperazin.

## Patentansprüche

1. Verfahren zur Herstellung eines Ethylenamingemisches, wobei ein Aminonitrilgemisch enthaltend mindestens 2 α-Aminonitrile zu jeweils mindestens 5 Gew.-% in Gegenwart eines Katalysators und gegebenenfalls einem Lösungsmittel hydriert wird.

2. Verfahren gemäß Anspruch 1, **dadurch gekennzeichnet, dass** ein Raney-Katalysator, insbesondere ein Raney-Nickel- oder ein Raney-Kobalt-Katalysator eingesetzt wird.

3. Verfahren gemäß Anspruch 1 oder 2, **dadurch gekennzeichnet, dass** die Hydrierung in Gegenwart von Wasser und/oder einem organischen Lösungsmittel, insbesondere Tetrahydrofuran oder Methanol, durchgeführt wird.

4. Verfahren gemäß einem der Ansprüche 1 bis 3, **dadurch gekennzeichnet, dass** das α-Aminonitril ausgewählt ist aus Aminoacetonitril (AAN), Iminodiacetonitril (IDAN), Ethylendiamindiacetonitril (EDDN), Ethylendiaminmonoacetonitril (EDMN), Diethylentriamindiacetonitril (DETDN), Diethylentriaminmonoacetonitril (DETMN) Piperazinylethylaminoacetonitril (PEAN), Aminoethylpiperazinylacetonitril (AEPAN) und Cyanomethylpiperazinylethylaminoacetonitril (CMPEAN).

5. Verfahren gemäß einem der Ansprüche 1 bis 4, **dadurch gekennzeichnet, dass** der Druck 30 bis 250 bar und/oder die Temperatur 80°C bis 140°C beträgt.

6. Verfahren gemäß einem der Ansprüche 1 bis 5, **dadurch gekennzeichnet, dass** im Ethylenamingemisch mindestens ein Ethylenamin enthalten ist ausgewählt aus Ethylendiamin (EDA), Diethylentriamin (DETA), Triethylentetraamin (TETA), Tetraethylenpentaamin (TEPA), Pentaethylenhexaamin (PEHA), Piperazin (Pip) oder Aminoethylpiperazin (AEPip).

7. Verfahren gemäß Anspruch 6, **dadurch gekennzeichnet, dass** eines oder mehrere der gebildeten Ethylenamine aus dem Ethylenamingemisch isoliert werden.

8. Verfahren gemäß einem der Ansprüche 1 bis 7, **dadurch gekennzeichnet, dass** das Aminonitrilgemisch mit einer Rate der Hydrierung zugeführt wird, die nicht größer ist als die Rate, mit der das Aminonitrilgemisch mit Wasserstoff bei der Hydrierung reagiert.

9. Verfahren gemäß einem der Ansprüche 1 bis 8, **dadurch gekennzeichnet, dass** die im Aminonitrilgemisch enthaltenen α-Aminonitrile durch Umsetzung von Formaldehydcyanhydrin (FACH) mit NH₃, EDA, DETA oder AEPip hergestellt werden.

10. Verfahren gemäß einem der Ansprüche 1 bis 9, **dadurch gekennzeichnet, dass** bei der Hydrierung hergestelltes DETA, EDA und/oder AEPip ganz oder teilweise zurückgeführt wird, um im Aminonitrilgemisch enthaltene α-Aminonitrile herzustellen.

11. Verfahren gemäß einem der Ansprüche 1 bis 10, **dadurch gekennzeichnet, dass** vor der Hydrierung aus dem Aminonitrilgemisch Leichtsieder abgetrennt werden oder dass gegebenenfalls zur Herstellung von α-Aminonitrilen FACH verwendet wird, aus dem die Leichtsieder abgetrennt worden sind.

12. Verfahren gemäß einem der Ansprüche 1 bis 11, **dadurch gekennzeichnet, dass** ein Aminonitrilgemisch hydriert wird enthaltend mindestens zwei der Komponenten a) bis e) mit
a) a) 10 bis 75 Gew.-% AAN,
b) 10 bis 50 Gew.-% IDAN, EDMN oder ein Gemisch davon,
c) 10 bis 70 Gew.-% EDDN, DETMN oder ein Gemisch davon, und
d) 5 bis 50 Gew.-% DETDN, und
e) 0 bis 10 Gew.-% PEAN, AEPAN, CMPEAN oder ein Gemisch davon.

## Claims

1. A process for preparing an ethylene amine mixture, which comprises hydrogenating an amino nitrile mixture comprising at least two α-amino nitriles in an amount of at least 5% by weight in each case in the presence of a catalyst and, if appropriate, a solvent.

2. The process according to claim 1, wherein a Raney catalyst, in particular a Raney nickel catalyst or a Raney cobalt catalyst, is used.

3. The process according to claim 1 or 2, wherein the hydrogenation is carried out in the presence of water and/or an organic solvent, in particular tetrahydrofuran or methanol.

4. The process according to any of claims 1 to 3, wherein the α-amino nitrile is selected from among aminoacetonitrile (AAN), iminodiacetonitrile (IDAN), ethylenediaminediacetonitrile (EDDN), ethylenediaminemonoacetonitrile (EDMN), diethylenetriaminediacetonitrile (DETDN), diethylenetriaminemonoacetonitrile (DETMN), piperazinylethylaminoacetonitrile (PEAN), aminoethylpiperazinylacetonitrile (AEPAN) and cyanomethylpiperazinylethylaminoacetonitrile (CMPEAN).

5. The process according to any of claims 1 to 4, wherein the pressure is from 30 to 250 bar and/or the temperature is from 80°C to 140°C.

6. The process according to any of claims 1 to 5, wherein the ethylene amine mixture comprises at least one ethylene amine selected from among ethylenediamine (EDA), diethylenetriamine (DETA), triethylenetetramine (TETA), tetraethylenepentamine (TEPA), pentaethylenehexamine (PEHA), piperazine (Pip) and aminoethylpiperazine (AEPip).

7. The process according to claim 6, wherein one or more of the ethylene amines formed is/are isolated from the ethylene amine mixture.

8. The process according to any of claims 1 to 7, wherein the amino nitrile mixture is fed into the hydrogenation at a rate which is no greater than the rate at which the amino nitrile mixture reacts with hydrogen in the hydrogenation.

9. The process according to any of claims 1 to 8, wherein the α-amino nitriles comprised in the amino nitrile mixture are prepared by reaction of formaldehyde cyanohydrin (FACH) with NH₃, EDA, DETA or AEPip.

10. The process according to any of claims 1 to 9, wherein DETA, EDA and/or AEPip produced in the hydrogenation is/are completely or partly recirculated in order to prepare α-amino nitriles comprised in the amino nitrile mixture.

11. The process according to any of claims 1 to 10, wherein low boilers are separated off from the amino nitrile mixture before the hydrogenation or FACH from which the low boilers have been separated off is, if appropriate, used for the preparation of α-amino nitriles.

12. The process according to any of claims 1 to 11, wherein an amino nitrile mixture comprising at least two of the components a) to e) in the following amounts:
a) from 10 to 75% by weight of AAN,
b) from 10 to 50% by weight of IDAN, EDMN or a mixture thereof,
c) from 10 to 70% by weight of EDDN, DETMN or a mixture thereof and
d) from 5 to 50% by weight of DETDN, and
e) from 0 to 10% by weight of PEAN, AEPAN, CMPEAN or a mixture thereof,
is hydrogenated.

## Revendications

1. Procédé pour la préparation d'un mélange d'éthylène-amines, où un mélange d'aminonitriles contenant au moins 2 α-aminonitriles à chaque fois à raison d'au moins 5% en poids est hydrogéné en présence d'un catalyseur et le cas échéant d'un solvant.

2. Procédé selon la revendication 1, **caractérisé en ce qu'**on utilise un catalyseur de Raney, en particulier un catalyseur de Raney à base de nickel ou un catalyseur de Raney à base de cobalt.

3. Procédé selon la revendication 1 ou 2, **caractérisé en ce que** l'hydrogénation est réalisée en présence d'eau et/ou d'un solvant organique, en particulier le tétrahydrofuranne ou le méthanol.

4. Procédé selon l'une quelconque des revendications 1 à 3, **caractérisé en ce que** l'a-aminonitrile est choisi parmi l'aminoacétonitrile (AAN), l'iminodiacétonitrile (IDAN), l'éthylènediaminodiacétonitrile (EDDN), l'éthylènediaminomonoacétonitrile (EDMN), le diéthylènetriaminodiacétonitrile (DETDN), le diéthylènetriaminomonoacétonitrile (DETMN), le pipérazinyléthylaminoacétonitrile (PEAN), l'aminoéthylpipérazinylacétonitrile (AEPAN) et le cyanométhylpipérazinyléthylaminoacétonitrile (CMPEAN).

5. Procédé selon l'une quelconque des revendications 1 à 4, **caractérisé en ce que** la pression est de 30 à 250 bars et/ou la température est de 80°C à 140°C.

6. Procédé selon l'une quelconque des revendications 1 à 5, **caractérisé en ce qu'**au moins une éthylène-amine, choisie parmi l'éthylènediamine (EDA), la diéthylènetriamine (DETA), la triéthylènetétraamine (TETA), la tétraéthylènepentaamine (TEPA), la pentaéthylènehexaamine (PEHA), la pipérazine (Pip) ou l'aminoéthylpipérazine (AEPip) est contenue dans le mélange d'éthylène-amines.

7. Procédé selon la revendication 6, **caractérisé en ce qu'**on isole une ou plusieurs des éthylène-amines formées du mélange d'éthylène-amines.

8. Procédé selon l'une quelconque des revendications 1 à 7, **caractérisé en ce que** le mélange d'aminonitriles est ajouté à une vitesse de l'hydrogénation qui n'est pas supérieure à la vitesse à laquelle le mélange d'aminonitriles réagit avec l'hydrogène lors de l'hydrogénation.

9. Procédé selon l'une quelconque des revendications 1 à 8, **caractérisé en ce que** les α-aminonitriles contenus dans le mélange d'aminonitriles sont préparés par transformation de formaldéhydecyanhydrine (FACH) avec NH₃, EDA, DETA ou AEPIp.

10. Procédé selon l'une quelconque des revendications 1 à 9, **caractérisé en ce que** la DETA, l'EDA et/ou l'AEPip préparée(s) lors de l'hydrogénation est/sont recyclée(s) totalement ou partiellement pour préparer des α-aminonitriles contenus dans le mélange d'aminonitriles.

11. Procédé selon l'une quelconque des revendications 1 à 10, **caractérisé en ce** des substances de bas point d'ébullition sont séparées avant l'hydrogénation du mélange d'aminonitriles ou en ce qu'on utilise le cas échéant pour la préparation des α-aminonitriles de la FACH, dont les substances de bas point d'ébullition ont été séparées.

12. Procédé selon l'une quelconque des revendications 1 à 11, **caractérisé en ce qu'**on hydrogène un mélange d'aminonitriles, contenant au moins deux des composants a) à e) avec
a) 10 à 75% en poids d'AAN,
b) 10 à 50% en poids d'IDAN, d'EDMN ou un mélange de ceux-ci,
c) 10 à 70% en poids d'EDDN, De DETMN ou un mélange de ceux-ci, et
d) 5 à 50% en poids de DETDN et
e) 0 à 10% en poids de PEAN, d'AEPAN, de CMPEAN ou un mélange de ceux-ci.
